# EUROPÄISCHE PATENTANMELDUNG

(11) **EP 0 615 763 A2**
(43) Veröffentlichungstag der Anmeldung: **21.09.1994**
(21) Anmeldenummer: 94103918.2
(22) Anmeldetag: 14.03.1994
(51) Int. Cl.: A61M 5/44, H05B 3/00, B01F 11/00

(54) **Aufwärm- und Auftaugerät**

(30) Priorität: 16.03.1993 DE 4308360
(71) Anmelder: TRANSMED Medizintechnik GmbH, D-33181 Wünnenberg (DE)
(72) Erfinder: Alt, Erwin, D-8098 Pfaffing (DE); Rappert, Klaus-Jürgen, D-33181 Wünnenberg (DE)
(74) Vertreter: Kehl, Günther, Dipl.-Phys.

(57) **Zusammenfassung**

Beschrieben ist ein Aufwärm- und Auftaugerät für in Behältnissen aufbewahrte medizinische Infusions- und Transfusionslösungen, mit einer Haltevorrichtung (6), die die Behältnisse (7) aufnimmt und während des Aufwärm- oder Auftauvorganges in Bewegung hält. Durch die Verwendung eines Infrarotstrahlers als Strahlungsquelle wird ein rasches und gleichzeitig schonendes Auftauen gewährleistet.

## Beschreibung

Die Erfindung bezieht sich auf ein Aufwärm- und Auflaugerät für in Behältnissen aufbewahrte medizinische Infusions- und Transfusionslösungen, mit einer Haltevorrichtung, die die Behältnisse aufnimmt und während des Aufwärm- oder Auftauvorganges in Bewegung hält, und mit einer Strahlungsquelle.

Tiefgefrorene Infusions- und Transfusionslösungen, wie beispielsweise gefrorenes Blutplasma, müssen schnell und schonend aufgetaut werden, um im Notfall oder bei einer Operation zur Verfügung zu stehen. Je schneller der Auftauvorgang durchgeführt werden kann, um so kleiner kann der Vorrat an im aufgetauten Zustand bereitgehaltenen Lösungen gehalten werden.

Es sind daher Aufwärm- und Auftaugeräte der eingangs genannten Art bekannt geworden (DE-OS 24 18 155), mit denen tiefgefrorene Lösungen mittels Mikrowellen aufgetaut und/oder auf Infusionstemperatur angewärmt werden können. Solche Geräte weisen jedoch Gefahr auf, daß sich infolge stehender Wellen in dem Mikrowellenresonator Stellen mit zu hoher Temperatur bilden, an denen beim Auftauen von Blutkonserven Bestandteile des Bluts geschädigt werden können. Außerdem sind Mikrowellengeräte aufwendig und teuer.

Auch Aufwärm- und Auftaugeräte mit Wasserbadbehältern, bei denen die Behältnisse mit den aufzutauenden Lösungen in ein erwärmtes Wasserbad eingehängt werden, sind bekannt (EP A1 0 432 591). Die Handhabung von Wasserbadbehältern ist jedoch aufwendig und zeitraubend.

Der Erfindung liegt daher die Aufgabe zugrunde, ein Aufwärm- und Aufgerät der eingangs genannten Art zu schaffen, das unter Beibehaltung des Vorteiles des raschen Aufwärmens sich vorallem dadurch auszeichnet, daß lokale Überhitzungen des Auftaugutes vermieden werden und somit ein schonendes Auftauen und/oder Aufwärmen möglich ist.

Diese Aufgabe wird dadurch gelöst, daß die Strahlungsquelle mindestens einen Infrarotstrahler aufweist.

Die gekühlten oder tiefgekühlten Infusions- und Transfusionslösungen befinden sich meist in Kunststoffbeuteln, die für Infrarotstrahlung gut durchlässig sind. Die Infrarotstrahlung kann daher direkt an das Aufwärm- bzw. Auftaugut, bei dem es sich um frisch gefrorenes Plasma oder um ein Erythrozytenkonzentrat handeln kann, gelangen. Da das Gut einen hohen Absorptionsfaktor für Infrarotstrahlung aufweist, wird es direkt durch die sich innerhalb des Gutes in Wärme umwandelnde Infrarotstrahlung erwärmt. Auf die Form der häufig unebenen oder ungleichmäßig geformten tiefgefrorenen Infusions- und Transfusionslösungen kommt es nicht an, da - wie erwähnt - die Wärme durch Strahlung in das Auftaugut gebracht wird.

Es haben sich Infrarotstrahler als besonders vorteilhaft erwiesen, bei denen der Hauptstrahlungsbereich im Wellenlängenbereich zwischen 600 nm und 1800 nm liegt.

Vorzugsweise werden solche Infrarotstrahler verwendet, bei denen die maximale Strahlungsemission bei 1200 nm liegt.

Nach einer vorteilhaften Ausführungsform der Erfindung ist eine Haltevorrichtung vorgesehen, die eine hin- und hergehende Bewegung, vorzugsweise eine Drehbewegung ausführt. Durch die permanente Bewegung der Haltevorrichtung und die dadurch verursachte Bewegung des Auftau- bzw. des Aufwärmegutes ist eine fortlaufende Durchmischung des Auftau- bzw. des Aufwärmgutes gewährleistet, sodaß örtliche Überhitzungen sicher vermieden werden.

Nach einer weiteren vorteilhaften Ausführungsform der Erfindung ist an der Haltevorrichtung eine Infrarotschattenmaske angeordnet. Durch die Infrarotschattenmaske können solche Bereiche des Behältnisses abgedeckt und vor der Infrarotstrahlung geschützt werden, in denen keine ausreichende Umwälzung der Lösungen gesichert ist, wie beispielsweise Anschlußschläuche oder dergleichen.

Vorzugsweise weist die Haltevorrichtung einen oder zwei Temperatursensoren auf, die so angeordnet sind, daß sie mit dem die medizinische Lösung enthaltenden Behältnis in Berührung kommen. Über den Temperatursensor oder die Temperatursensoren kann somit die Temperatur des Behältnisinhalts ermittelt werden, da der auf den Temperatursensor abgeleitete Wärmestrom ein direktes Maß für die Temperatur der Lösung ist.

Der Temperatursensor oder die beiden Temperatursensoren sind vorzugsweise durch einen Reflektor vor direkter Bestrahlung durch den Infrarotstrahler geschützt, sodaß sichergestellt ist, daß der Temperatursensor nur durch Wärmeleitung aus dem Behältnisinhalt erwärmt wird und eine korrekte Temperaturmessung gewährleistet ist.

Eine besonders schonende Behandlung von Blutkonserven und Erythrozytenkonzentraten ist nach einer vorteilhaften Weiterentwicklung der Erfindung dann gewährleistet, wenn eine Filterplatte vorgesehen wird, die für Infrarotstrahlung im Wellenlängenbereich oberhalb von 1400 nm absorbierend und im Wellenlängenbereich von 780 bis 1400 nm durchlässig ist.

Durch einen vor dem Infrarotstrahler angeordneten Rotfilter, der Strahlung mit einer Wellenlänge unterhalb von 600 nm ausfiltert, wird - nach einer weiteren vorteilhaften Ausführungsform - die Hämolyse bei Blutkonserven oder Erythrozytenkonzentraten wirksam verhindert.

Durch eine Ventilatoreinrichtung kann erwärmte Luft abgeführt werden, die zu einem Wärmestau und somit zu einer Überhitzung des Auftau/Aufwärmgutes führen könnte.

Nach einer weiteren vorteilhaften Ausführungsform ist ein Sensor vorgesehen, der die ständige Bewegung der Haltevorrichtung überprüft. Bei einem Ausfall der Bewegung der Haltevorrichtung kann das Ausgangssignal des Sensors zum Abschalten der Strahlungsquelle verwendet werden.

Durch eine Steuereinrichtung, die mit einer Antriebseinrichtung für die Haltevorrichtung, einem Temperatursensor, dem Infrarotstrahler und einem die Bewegung der Haltevorrichtung erfassenden Sensor verbunden ist, kann der Aufwärm- und Auftauvorgang automatisch überwacht werden. Durch einen Sensor für den Schließzustand der Beladetür, der ebenfalls mit der Steuereinrichtung verbunden sein kann, kann die Stromzufuhr zu dem Infrarotstrahler unter-brochen werden, wenn die Beladetür geöffnet wird. Dadurch entsteht ein Höchstmaß an Sicherheit für den Betreiber hinsichtlich mechanischer Verletzungen und Schäden durch die Strahlung.

Die Erfindung wird im folgenden anhand eines in den Figuren schematisch dargestellten Ausführungsbeispieles näher erläutert.

Es zeigt:
- Figur 1:: Ein Aufwärm- und Auftaugerät im Längsschnitt.
- Figur 2:: Einen Schnitt längs der Schnittlinie II-II der Figur 1.
- Figur 3:: Ein Prinzipschaltbild für das Auftau- und Aufwärmgerät.

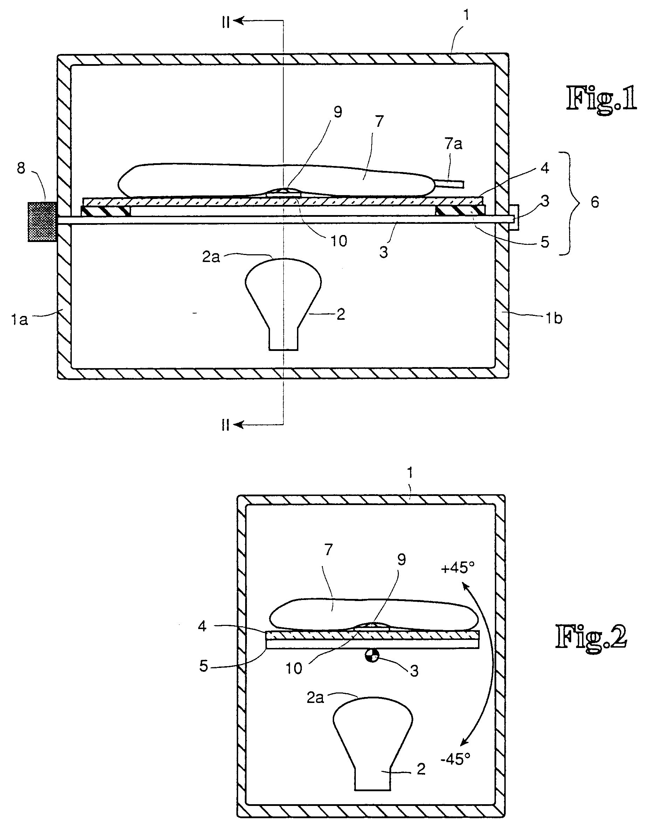

Das Auftau- und Aufwärmgerät, wie es in den Figuren 1 und 2 gezeigt ist, weist ein etwa quaderförmiges Gehäuse 1 auf. An dem Gehäuse ist eine Zugangstür angeordnet, die jedoch in der Zeichnung nicht zu erkennen ist. Im Bodenbereich des Gehäuses 1 ist in zentraler Lage ein Infrarotstrahler 2 angeordnet, der senkrecht nach oben gerichtet ist. An zwei gegenüberliegenden Seitenwänden 1a und 1b des Gehäuses 1 ist eine Welle 3 drehbar gelagert. An der Welle 3 ist über einen (in der Zeichnung nicht zu erkennenden Rahmen) eine horizontale Filterplatte 4 mit einer Schattenmaske 5 befestigt.

Die Welle 3 sowie die an ihr befestigte Filterplatte 4 mit der Schattenmaske 5 bilden zusammen eine Haltevorrichtung 6 für ein Behältnis 7, das eine medizinische Lösung enthält. Es können Befestigungsmittel vorgesehen werden, mit denen das Behältnis an der Filterplatte 4 festgeschnallt oder festgeklemmt wird. Diese sind in der Zeichnung jedoch nicht gezeigt. Die Welle 3 ist mit einer an der Außenseite des Gehäuses 1 angeordneten elektrischen Antriebseinrichtung 8 verbunden, durch die die Welle 3 in eine hin-und hergehende Drehbewegung versetzt wird. An der Oberseite der Filterplatte 4 ist ein Temperatursensor 9 angeordnet, auf dem das Behältnis 7 aufliegt. Durch einen Reflektor 10 ist der Temperatursensor 9 vor direkter Bestrahlung durch den Infrarotstrahler 2 geschützt, sodaß er nur auf Wärme anspricht, die durch Wärmeleitung aus dem Behältnis 7 auf ihn übertragen wird.

Die Filterplatte 4 ist für Strahlung im Wellenlängenbereich von 780 bis 1400 nm durchlässig (IR-A und IR-B-Strahlung), während Strahlung größerer Wellenlänge (IR-C-Strahlung) absorbiert wird. Der Infrarotstrahler 2 ist an seiner Strahlungsaustrittseite mit einem Rotfilter 2a versehen, das Strahlung ausfiltert, deren Wellenlänge unterhalb von 600 nm liegt. Die aus dem Infrarotstrahler 2 austretende und durch das Rotfilter 2a sowie durch die Filterplatte 4 durchtretende Strahlung gewährleistet eine rasche und schonende Erwärmung des in dem Behältnis 7 befindlichen Gutes. Durch die Schattenmaske 5 wird die Strahlung vom Randbereich der Filterplatte 4 gänzlich ferngehalten, in dem sich ein Anschlußschlauch 7a des Behältnisses 7 befindet.

Beim Betrieb des Gerätes wird durch die Antriebseinrichtung 8 die Welle 3 in eine hin- und hergehende Bewegung versetzt, sodaß die Haltevorrichtung 6 und das auf der Haltevorrichtung angeordnete Behältnis 7 eine Schwenkbewegung ausführen, die sich von +45° bis -45° gegenüber der in den Zeichnungen gezeigten horizontalen Mittellage erstreckt.

Diese Bewegung kann sehr langsam und schonend ausgeführt werden, sodaß keine mechanische Beeinträchtigung der Erythrozyten in dem Behältnis 7 zu befürchten ist (falles es sich bei dem Inhalt um Blut oder ein Erythrozytenkonzentrat handelt). Gleichwohl ist eine Druchmischung des Inhalts des Behältnisses 7 gewährleistet und somit die Gefahr einer lokalen Überhitzung verhindert.

Das Gehäuse ist mit Lüftungsschlitzen und einem Ventilator versehen (in den Zeichnungen nicht zu erkennen) durch die Warmluft aus dem Gehäuse 1 abgeführt und Frischluft angesaugt wird, um einen Wärmestau, insbesondere nach dem Abschalten des Infrarotstrahlers 2 zu vermeiden.

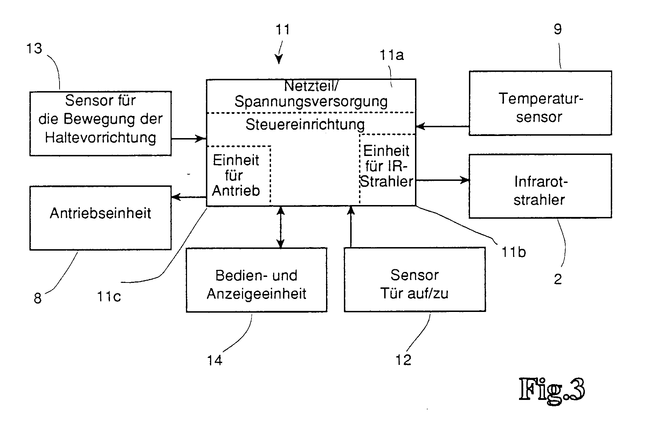

Die Funktionsweise des Aufwärm- und Auftaugerätes wird anhand des Blockschaltbildes der Figur 3 erläutert. In Figur 3 ist eine Steuereinrichtung 11 zu erkennen, die ein Netzteil 11a, sowie eine Steuereinheit 11b für den Infrarotstrahler 2 und eine Steuereinheit 11c für die Antriebseinrichtung 8 aufweist. Mit der Steuereinrichtung 11 ist der Temperatursensor 9, ein Sensor 12 für den Schließzustand der Beladetür, ein Sensor 13 für die Bewegung der Haltevorrichtung 6 sowie eine Bedien- und Anzeigeeinheit 14 verbunden.

Wenn das Gerät über die Bedien- und Anzeigeeinheit 14 in Betrieb gesetzt wird, wird von der Steuereinrichtung 11 die Antriebseinrichtung 8 eingeschaltet, sodaß die Welle 3 in eine hin- und hergehende Drehbewegung versetzt wird. Gleichzeitig wird der Infrarotstrahler 2 in Betrieb genommen.

Durch den Sensor 13 wird die hin- und hergehende Bewegung der Welle 3 überwacht. Falls die Welle 3 zum Stillstand kommt während das Gerät in Betrieb ist, wird der Infrarotstrahler 2 sofort abgeschaltet. Eine umgehende Abschaltung erfolgt auch, wenn der Temperatursensor 9 an die Steuereinrichtung 11 eine über dem Sollwert von beispielsweise 37° C liegende Temperatur meldet.

## Patentansprüche

1. Aufwärm- und Auftaugerät für in Behältnissen aufbewahrte medizinische Infusions- und Transfusionslösungen, mit einer Haltevorrichtung (6), die die Behältnisse (7) aufnimmt und während des Aufwärm- oder Auftauvorganges in Bewegung hält, und mit einer Strahlungsquelle, dadurch **gekennzeichnet**, daß die Strahlunsgquelle mindestens einen Infrarotstrahler (2) aufweist.

2. Aufwärm- und Auttaugerät nach Anspruch 1, dadurch gekennzeichnet, daß der Hauptstrahlungsbereich des Infrarotstrahlers (2) im Wellenlängenbereich zwischen 600 nm und 1800 nm liegt.

3. Aufwärm- und Auftaugerät nach Anspruch 1 oder 2, dadurch gekennzeichnet, daß die maximale Strahlungsemission des Infrarotstrahlers bei 1200 nm liegt.

4. Aufwärm- und Auftaugerät nach einem der Ansprüche 1 bis 3, dadurch gekennzeichnet, daß die Haltevorrichtung (6) eine hin- und hergehende Bewegung ausführt.

5. Aufwärm- und Auftaugerät nach Anspruch 4, dadurch gekennzeichnet, daß die hin- und hergehende Bewegung der Haltevorrichtung (6) eine Drehbewegung ist.

6. Aufwärm- und Auftaugerät nach einem der Ansprüche 1 bis 5, dadurch gekennzeichnet, daß an der Haltevorrichtung (6) eine Infrarot-Schattenmaske (5) angeordnet ist.

7. Aufwärm- und Auftaugerät nach einem der Ansprüche 1 bis 6, dadurch gekennzeichnet, daß an der Haltevorrichtung (6) mindestens ein Temperatursensor (9) angeordnet ist, der mit dem an der Haltevorrichtung (6) befestigten Behältnis (7) in Berührung kommt.

8. Aufwärm- und Auftaugerät nach Anspruch 7, dadurch gekennzeichnet, daß an der dem Infrarotstrahler (2) zugewandten Seite des Temperatursensors (9) ein Reflektor (10) angeordnet ist.

9. Aufwärm- und Auftaugerät nach einem der Ansprüche 1 bis 8, dadurch gekennzeichnet, daß eine für Infrarotstrahlung im Wellenlängenbereich oberhalb von 1400 nm absorbierende und im Wellenbereich von 780 bis 1400 nm durchlässige Filterplatte (4) an der zum Infrarotstrahler (2) weisenden Seite des Behältnisses (7) angeordnet ist.

10. Aufwärm- und Auftaugerät nach einem der Ansprüche 1 bis 9, gekennzeichnet durch einen vor dem Infrarotstrahler (2) angeordnetes Rotfilter (2a), das Strahlung mit einer Wellenlänge unterhalb von 600 nm ausfiltert.

11. Aufwärm- und Auftaugerät nach einem der Ansprüche 1 bis 10, gekennzeichnet durch eine Ventilatoreinrichtung.

12. Aufwärm- und Auftaugerät nach einem der Ansprüche 1 bis 11, gekennzeichnet durch einen die Bewegung der Haltevorrichtung (6) erfassenden Sensor (13).

13. Aufwärm- und Auftaugerät nach einem der Ansprüche 1 bis 12, gekennzeichnet durch eine Steuereinrichtung (11), die mit einer Antriebseinrichtung (8) für die Haltevorrichtung (6), einem Temperatursensor (9), dem Infrarotstrahler (2) und einem die Bewegung der Haltevorrichtung (6) erfassenden Sensor (13) verbunden ist.

14. Aufwärm- und Auftaugerät nach Anspruch 13, dadurch gekennzeichnet, daß die Steuereinrichtung (11) mit einem Sensor (12) für den Schließzustand der Beladetür verbunden ist.

15. Aufwärm- und Auftaugerät nach Anspruch 13 oder 14, dadurch gekennzeichnet, daß die Steuereinheit (11) mit einer Bedien- und Anzeigeeinheit (14) verbunden ist.
